# EUROPEAN PATENT SPECIFICATION

(11) **EP 2 394 582 B1**
(45) Date of publication and mention of the grant of the patent: **12.12.2012**
(21) Application number: 10829800.1
(22) Date of filing: 13.10.2010
(51) Int. Cl.: A61B 8/12, A61B 8/08

(54) **ULTRASOUND OBSERVATION APPARATUS**
ULTRASCHALL-BEOBACHTUNGSGERÄT
DISPOSITIF D'OBSERVATION ULTRASONORE

(30) Priority: 16.11.2009 JP 2009261153
(43) Date of publication of application: 14.12.2011
(73) Proprietor: OLYMPUS MEDICAL SYSTEMS CORP., Tokyo 151-0072 (JP)
(72) Inventor: MIYAKI, Hironaka, Tokyo 151-0072 (JP); ECHIZENYA, Takahiro, Tokyo 151-0072 (JP); OTANI, Shuji, Tokyo 151-0072 (JP)
(74) Representative: Gunzelmann, Rainer
(86) International application number: PCT/JP2010/067950
(87) International publication number: WO 2011/058840

(56) References cited:
- EP-A1- 1 629 778
- EP-A1- 2 036 500
- JP-A- 2000 185 041
- JP-A- 2005 058 584
- JP-A- 2005 342 128
- JP-A- 2006 175 006
- JP-A- 2007 175 431
- JP-A- 2009 118 961
- JP-A- 2009 254 780
- US-A1- 2004 106 869
- US-A1- 2010 063 401

## Description

### Technical Field

The present invention relates to an ultrasound observation apparatus for generating a B-mode image of inside a subject by scanning an ultrasound beam on a predetermined scanning plane in the subject.

### Background Art

As disclosed in a patent document 1, for example, an ultrasound observation apparatus used in a medical field and the like is provided with an ultrasound probe capable of transmitting and receiving ultrasound to and from a subject and generates a B-mode image as a cross-sectional image of a subject. The B-mode image is acquired by scanning an ultrasound beam on a predetermined scanning plane.

As disclosed in the Japanese Patent Application Laid-Open Publication No. 2006-175006, using such an ultrasound observation apparatus makes it possible to perform a treatment while checking on a B-mode image a position and a posture of a treatment instrument such as a puncturing needle, a biopsy forceps, or cytological brush with respect to a predetermined region in a subject.

When performing a treatment with respect to a predetermined region in a subject using a treatment instrument such as a puncturing needle, biopsy forceps or cytological brush, the treatment instrument is applied with a force, which deviates the position of the treatment instrument from the scanning plane and makes it difficult to observe the treatment instrument on the B-mode image in some cases. Such deviation of a treatment instrument from a scanning plane causes the need of a troublesome operation to change the position and orientation of the ultrasound probe to match the scanning plane with the treatment instrument.

Document EP 1 629 778 A1 concerns an ultrasonic endoscope by which a positional relationship can be grasped even in the width direction of ultrasonic transducers. The ultrasonic endoscope performs three-dimensional scanning operation by using an ultrasonic transducer array having plural two-dimensional ultrasonic transducer arrays arranged in a semicircle form, and includes plural signal interconnections, each for inputting the same drive signal to the individual electrodes of the ultrasonic transducers located in the same row and column in the plural two-dimensional ultrasonic transducer arrays, and plural common interconnections, each for connecting common electrodes of the plural ultrasonic transducers included in respective ones of the plural two-dimensional ultrasonic transducer arrays to a fixed potential.

The present invention was made in view of the above-described points and has an objective to provide an ultrasound observation apparatus which observes the situation of a treatment using a treatment instrument in a subject and which is capable of continuing an excellent observation of the treatment instrument, and a control method of the ultrasound observation apparatus.

### Disclosure of Invention

### Means for Solving the Problem

According to one aspect of the present invention, an ultrasound observation apparatus comprising the features of claim 1.

### Brief Description of the Drawings

FIG. 1 is a view showing a schematic configuration of an ultrasound observation apparatus according to a first embodiment.
FIG. 2 is a perspective view showing a detailed configuration of a distal end portion of an insertion portion of an ultrasound endoscope according to the first embodiment.
FIG. 3 shows a side surface part of the distal end portion of the insertion portion according to the first embodiment.
FIG. 4 is a view of the distal end portion of the insertion portion according to the first embodiment when viewed from a distal end direction of an insertion axis.
FIG. 5 is a view illustrating a configuration of an ultrasound observation control unit according to the first embodiment.
FIG. 6 is a view showing an example of a B-mode image in a case where a treatment instrument exists on a scanning plane according to the first embodiment.
FIG. 7 is a view showing an example of a sample image according to the first embodiment.
FIG. 8 is a flowchart describing an operation of the ultrasound observation control unit according to the first embodiment.
FIG. 9 is a flowchart of a scanning plane optimization process according to the first embodiment.
FIG. 10 is a view showing a state where an advancing direction of the treatment instrument according to the first embodiment is deviated from a central axis of a treatment instrument insertion port.
FIG. 11 is a view showing an example of a B-mode image in a case where the treatment instrument is deviated from the scanning plane according to the first embodiment.
FIG. 12 is a view showing a configuration of an ultrasound probe portion according to a second embodiment.
FIG. 13 is a view showing a configuration of an ultrasound observation control unit according to the second embodiment.

### Best Mode for Carrying Out the Invention

Hereinafter, preferred embodiments of the present invention will be described with reference to drawings. Note that, in the drawings used for description below, a different scale size is used for each of the components in order to allow each of the components to be illustrated in a recognizable size in the drawings, and the present invention is not limited to the number, the shapes, the ratio of the sizes of the components, and a relative positional relationship among the components shown in these drawings.

### (First Embodiment)

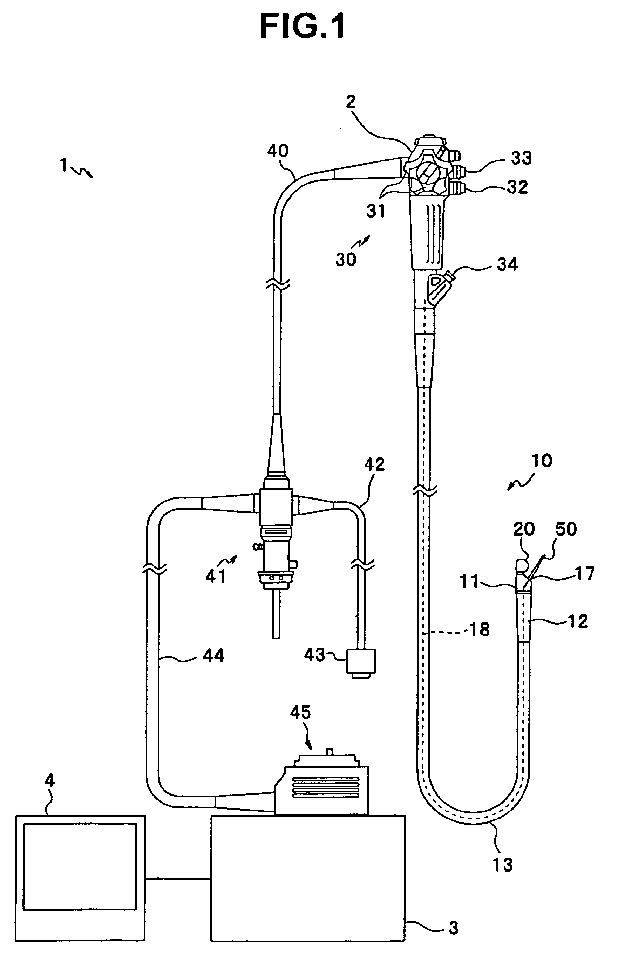

The first embodiment of the present invention will be described below. An ultrasound observation apparatus 1 according to the present embodiment shown in FIG. 1 includes an ultrasound endoscope 2 and an ultrasound observation control unit 3.

The ultrasound observation apparatus 1 is schematically an apparatus which generates a B-mode image (ultrasound tomographic image) of a predetermined region in a subject by scanning an ultrasound beam in the subject, to output the B-mode image to an image display apparatus 4.

The ultrasound endoscope 1 is configured by mainly including an insertion portion 10 which can be introduced in the subject, an operation portion 30 positioned at a proximal end of the insertion portion 10, a universal cord 40 which extends from a side portion of the operation portion 30.

The insertion portion 10 includes the following components in a linked manner: a distal end portion 11 disposed at a distal end of the insertion portion; a bendable bending portion 12 disposed at a proximal end side of the distal end portion 11, and a flexible tube portion 13 having flexibility which is disposed at a proximal end side of the bending portion 12 and connected to a distal end side of the operation portion 30. Though detailed description will be made later, the distal end portion 11 is provided with an ultrasound probe portion 20, a treatment instrument insertion port 17, a fluid feeding portion 14, an image pickup apparatus 15, an illumination device 16, and the like.

The insertion portion 10A includes inside thereof a treatment instrument insertion conduit 18. The treatment instrument insertion conduit 18 is a conduit for communicating the treatment instrument insertion port 17 as an opening portion provided at the distal end portion 10 with a conduit cap 34 provided at the operation portion 30.

The operation portion 30 is provided with an angle knob 31 for operating bending of the bending portion 12, an air-feeding/water-feeding button 32 for controlling a feeding operation of fluid from the fluid feeding portion 14 provided at the distal end portion 10, a suction button 33 for controlling a suction operation from the treatment instrument insertion port 17, the conduit cap 34, and the like.

An endoscope connector 41 to be connected to a light source device, not shown, is provided at a proximal end portion of the universal cord 40. The light emitted from the light source device passes through the universal cord 40, the operation portion 30, and an optical fiber cable inserted through the insertion portion 10, to be emitted from the illumination device 16 of the distal end portion 11. Note that the ultrasound endoscope 1 may be provided with a light source device such as an LED at the distal end portion 11.

An electric cable 42 and an ultrasound cable 44 are extended from the endoscope connector 41. The electric cable 42 is detachably connected to a camera control unit, not shown, through an electric connector 43. The camera control unit is electrically connected to the image pickup apparatus 15 provided at the distal end portion 11 through the electric cable 42. The camera control unit is electrically connected to the image display apparatus 4 and outputs an image picked up by the image pickup apparatus 15 to the image display apparatus 4.

The ultrasound cable 44 is detachably connected to the ultrasound observation control unit 3 to be detailed later, through an ultrasound connector 45.

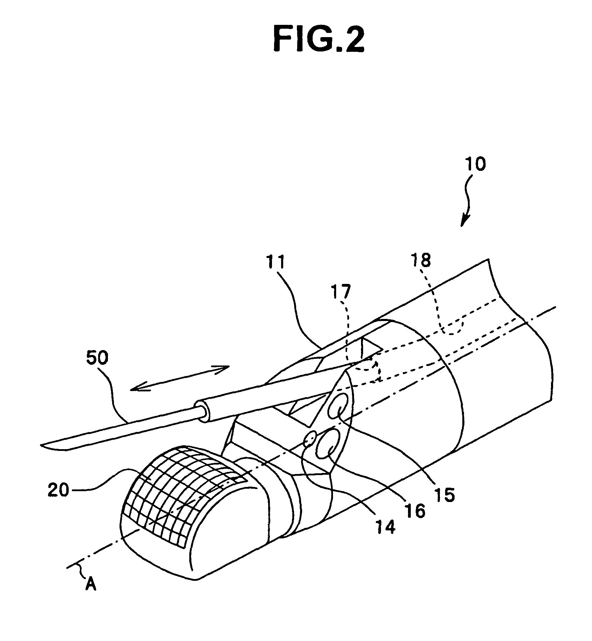

Next, description will be made on a detailed configuration of the distal end portion 11 of the insertion portion 10 of the ultrasound endoscope 2. As shown in FIG. 2, the distal end portion 11 includes the ultrasound probe portion 20, the treatment instrument insertion port 17, the image pickup apparatus 15, the illumination device 16, and the fluid feeding portion 14.

The ultrasound probe portion 20 is configured to be capable of changing the transmission/reception direction of the ultrasound beam in a first direction L1 and in a second direction L2. That is, the ultrasound probe portion 20 is configured so as to enable two-dimensional scanning of an ultrasound beam.

The configuration of the ultrasound probe portion 20 is not specifically limited as long as the two-dimensional scanning of the ultrasound beam is possible. In the present embodiment, as an example, the ultrasound probe portion 20 includes a plurality of ultrasound transducers which are aligned in matrix and can be individually driven, and is configured to electronically perform the two-dimensional scanning of the ultrasound beam by controlling a driving timing of each of the ultrasound transducers.

As the ultrasound transducers constituting the ultrasound probe portion 20, piezoelectric elements or electrostrictive elements such as piezoelectric ceramics, ultrasonic transducers by micromachine technology (MUT: Micromachined Ultrasonic Transducer), or the like can be applied, for example.

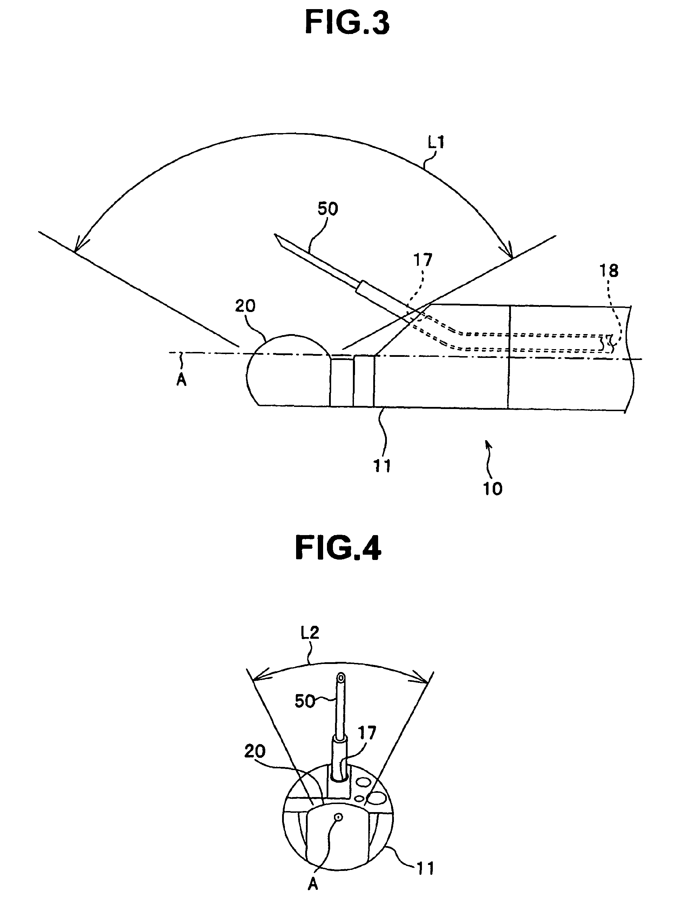

More specifically, the ultrasound probe portion 20 of the present embodiment can change the transmission/reception direction of the ultrasound beam in a substantially sector shape on a plane parallel to an insertion axis A of the insertion portion 10 to perform scanning of an ultrasound beam, as shown in FIG. 3. In the present embodiment, as shown by the arrow L1 in FIG. 3, the amplitude direction of the scanning of the ultrasound beam on the plane substantially parallel to the insertion axis A is defined as a first direction L1.

In addition, in the present embodiment, the plane including a central axis of the ultrasound beam which is scanned in the first direction L1 is referred to as a scanning plane, and the B-mode image generated by the ultrasound observation apparatus 1 is assumed to be acquired by scanning the ultrasound beam on the scanning plane.

As shown in FIG. 4, the ultrasound probe portion 20 of the present embodiment can change the transmission/reception direction of the ultrasound beam in a substantially sector shape on the plane perpendicular to the insertion axis A of the insertion portion 10 to perform scanning of the ultrasound beam. In the present embodiment, as shown by the arrow L2 in FIG. 4, the amplitude direction of the scanning of the ultrasound beam on the plane perpendicular to the insertion axis A is defined as a second direction L2.

That is, the ultrasound probe portion 20 of the present embodiment enables the scanning plane to move in the second direction L2 by changing the transmission/reception direction of the ultrasound beam in the second direction L2. In the present embodiment, as an example, the scanning plane can be moved to positions of a plurality of predetermined locations within the range of scanning in the second direction L2.

Note that the ultrasound probe portion 20 according to the present embodiment which is shown in drawings is configured by aligning the ultrasound transducers in a substantially circular arc shape along the first direction L1 and the second direction L2. However, it is needless to say that electronic two-dimensional scanning of the ultrasound beam is possible even if the plurality of ultrasound transducers are aligned in matrix on a plane.

The treatment instrument insertion port 17 is an opening portion for allowing a treatment instrument 50 to protrude therefrom, and communicates with the treatment instrument insertion conduit 18. In the ultrasound endoscope 2 according to the present embodiment, the treatment instrument 50 is inserted from the opening portion of the conduit cap 34 to allow the treatment instrument 50 to protrude from the treatment instrument insertion port 17 of the distal end portion 11, thereby enabling the treatment instrument 50 to be introduced into a body of a subject, for example.

Note that the type of the treatment instrument 50 is not specifically limited, but a puncture needle, a biopsy forceps, a cytological brush, or the like can be listed, for example. In the present embodiment, the treatment instrument 50 is a puncture needle, as shown in FIG. 2. The treatment instrument 50 may be provided with an ultrasound scattering portion for scattering ultrasound waves in order to make the echo pattern of the treatment instrument 50 in a B-mode image clearer.

Furthermore, in the present embodiment, the treatment instrument insertion port 17 is disposed such that the treatment instrument 50 protruded from the treatment instrument insertion port 17 advances in a range of scanning of the ultrasound beam performed by the ultrasound probe portion 20, as shown in FIGS. 3 and 4.

In other words, the ultrasound observation apparatus 1 according to the present embodiment is configured to be able to capture the treatment instrument 50 protruded from the treatment instrument insertion port 17 in a B-mode image by moving the scanning plane.

In addition, the present embodiment employs, as one example, a configuration in which, when the scanning plane is moved to the center of the range of scanning in the second direction L2, the scanning plane and the central axis of the treatment instrument insertion port 17 are positioned on substantially the same plane.

The image pickup apparatus 15 includes an image-forming optical system member and an image pickup device, and picks up an optical image. The image pickup apparatus 15 is disposed so as to capture the distal end direction along the insertion axis A within the field of view thereof. The illumination device 16 emits the light generated by the light source device into the field of view of the image pickup apparatus 15. The fluid feeding portion 17 is an opening portion provided at the distal end portion 11, and fluid is fed from the fluid feeding portion 17 by operating the air-feeding/water-feeding button 32 provided at the operation portion 30.

Next, description will be made on the detailed configuration of the ultrasound observation control unit 3. The ultrasound observation control unit 3 includes a calculation device, a storage device, an input/output device, a power control device and the like, and is a control apparatus which controls operation of the ultrasound probe portion 20 and generates and outputs a B-mode image based on a predetermined program.

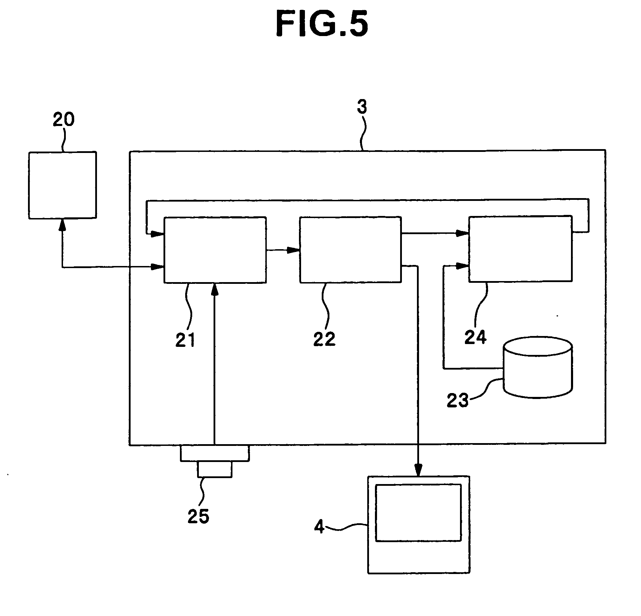

The ultrasound observation control unit 3 includes, as shown in FIG. 5, a transmission/reception control section 21, a B-mode image calculation section 22, a storage section 23, a correlation calculation section 24, and an ultrasound observation switch 25, as components required for achieving the operation to be described later of the ultrasound observation apparatus 1. Note that the transmission/reception control section 21, the B-mode image calculation section 22, and the correlation calculation section 24 may be mounted to the ultrasound observation control unit 3 either in a hardware manner or in a software manner.

The transmission/reception control section 21 controls the transmission/reception direction of the ultrasound beam transmitted and received by the ultrasound probe portion 20. That is, the transmission/reception control section 21 controls the position of the scanning plane with respect to the second direction L2 and the scanning of the ultrasound beam for acquiring the B-mode image on the scanning plane.

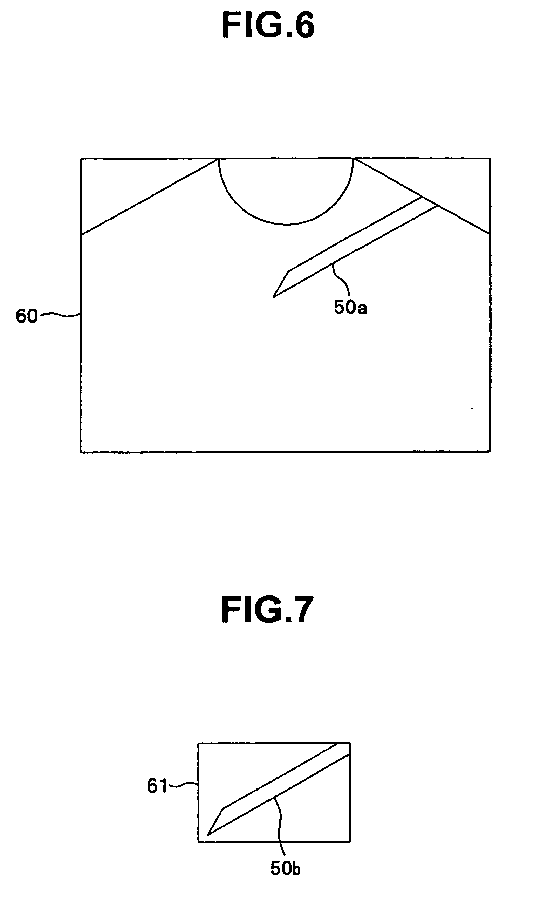

The B-mode image calculation section 22 generates the B-mode image on the scanning plane based on the result of scanning of the ultrasound beam performed by the ultrasound probe portion 20. When the treatment instrument 50 exists on the scanning plane, for example, an echo pattern 50a of the treatment instrument 50 appears in a B-mode image 60, as shown in FIG. 6.

The storage section 23 stores a predetermined sample image determined according to the shape of treatment instrument 50. Specifically, the sample image is an image showing a shape and a size of an ideal echo pattern of the treatment instrument 50 in the B-mode image in the case where the scanning plane and the central axis of the treatment instrument 50 agree with each other.

When the treatment instrument 50 is a puncture needle as in the present embodiment, a sample image 61 is an image showing a shape and a size of an echo pattern 50b of the distal end portion of the puncture needle in the case where the central axis of the puncture needle agrees with the scanning plane, as shown in FIG. 7.

The present embodiment describes, as an example, that the sample image 61 is created in advance according to the type and the shape of the treatment instrument 50. Note that the sample image 61 may be stored in the storage section 23 as a result of designation of the echo pattern 50a of the treatment instrument 50 in the actual B-mode image 60 by a user of the ultrasound observation apparatus 1, that is, may be stored as the echo pattern 50a of the treatment instrument 50 to be actually used, by what is called a teaching operation.

In addition, the shape of the treatment instrument 50 is hard to appear in the B-mode image 60 in some cases depending on a shape of the treatment instrument 50 or a material configuring the treatment instrument 50. However, in such a case, the ultrasound scattering portion is provided in a part of the treatment instrument 50. When the ultrasound scattering portion is provided in the treatment instrument 50, the sample image 61 is an image showing a shape of an ideal echo pattern of the ultrasound scattering portion in the B-mode image 60.

The correlation calculation section 24 calculates a correlation value R between the B-mode image 60 and the sample image 61. Specifically, the correlation calculation section 24 performs on the B-mode image 60 an image processing referred to as a pattern matching by using the sample image 61 as a template, and calculates a similarity between the echo pattern in the B-mode image 60 and the sample image 61. The higher the similarity between the echo pattern in the B-mode image 60 and the sample image 61, the higher the correlation value R. Since the pattern matching is a well-known technology, detailed description thereof will be omitted.

The ultrasound observation switch 25 is an input device through which a user inputs instructions for starting and terminating the observation using a B-mode image. In the present embodiment, the ultrasound observation switch 25 is provided to the ultrasound observation control unit 3, as an example. However, the ultrasound observation switch 25 may be provided to the operation portion 30 of the ultrasound endoscope 2, or may be configured as a switch like a foot switch provided separately from the ultrasound observation control unit 3 and the ultrasound endoscope 2.

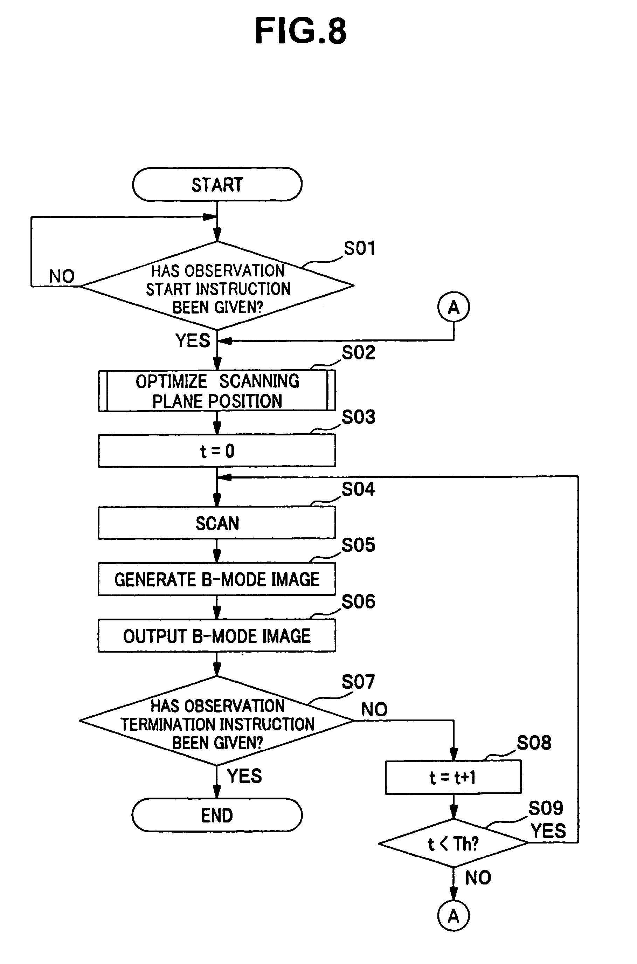

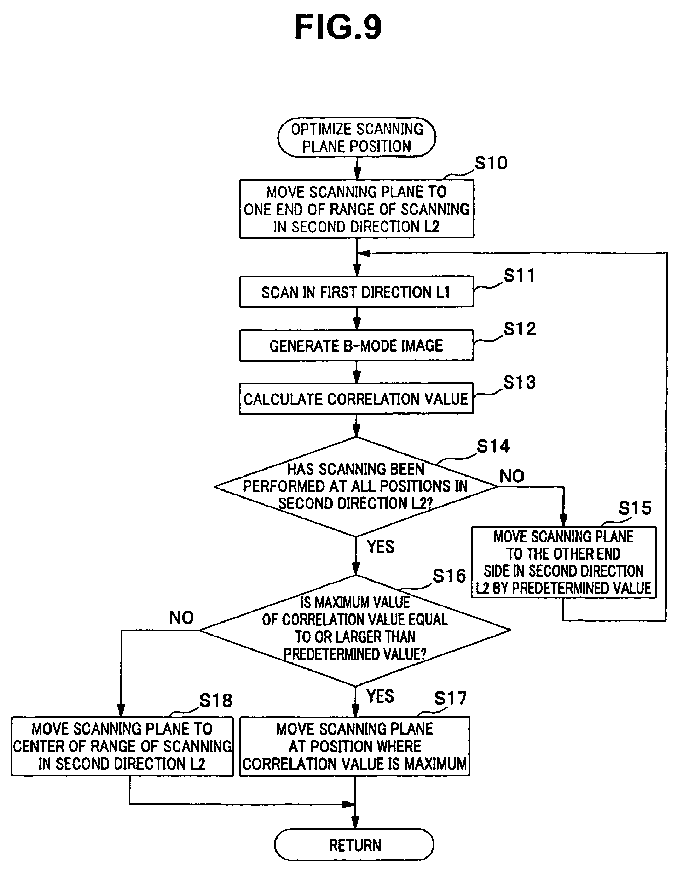

Next, the operation of the ultrasound observation apparatus 1 will be described with reference to the flowcharts in FIGS. 8 and 9. Note that description will be made below on the B-mode image generation operation performed by the ultrasound probe portion 20 and the ultrasound observation control unit 3, and description on the optical image observation operation performed by the image pickup apparatus 15 provided in the ultrasound endoscope 1 will be omitted.

First, in step S01, a stand-by state continues until an instruction for starting the observation using a B-mode image is inputted by operating the ultrasound observation switch 25. When the instruction for starting the observation using a B-mode image is inputted, the process moves to step S02, and the scanning plane position optimization process shown in the flowchart in FIG. 9 is performed.

In the scanning plane position optimization process, first in step S 10, the transmission/reception control section 21 moves the scanning plane to one end of the range of scanning in the second direction L2. Then, in step S 11, the transmission/reception control section 21 controls the ultrasound probe portion 20 to cause the ultrasound beam to scan in the first direction L1 on the scanning plane whose position with respect to the second direction L2 is determined.

Next, in step S12, the B-mode image calculation section 22 generates a B-mode image based on the result of scanning in the step S11. Then, in step S 13, the correlation calculation section 24 calculates a correlation value between the B-mode image acquired in the step S12 and the sample image stored in the storage section 23.

Next, in step S14, it is determined whether or not scanning has been performed at all positions determined in advance, regarding the position of the scanning plane with respect to the second direction L2. When scanning has not been performed at all the positions, regarding the second direction L2, the process moves to step S 15. In the step S 15, after the scanning plane is moved to the next position on the other end side in the second direction L2, the processes in the step S11 to step S13 are repeated.

Then, in the step S 14, when it is determined that scanning has been performed at all the positions determined in advance regarding the position of the scanning plane with respect to the second direction L2, the process moves to step S16.

That is, the steps S 10 to S 15 are the processes of acquiring a plurality of B-mode images with the scanning plane positioned at a plurality of locations in the second direction L2, and calculating correlation values between the plurality of B-mode images and the sample image.

In the step S16, it is determined whether or not the maximum value of the correlation values between the plurality of B-mode images acquired in the above process and the sample image is equal to or larger than a predetermined threshold. When the maximum value of the correlation values is equal to or larger than the predetermined threshold, the process moves to step S 17.

In step S 17, the transmission/reception control section 21 moves the position of the scanning plane with respect to the second direction L2 to a position where the maximum correlation value is acquired among the correlation values of the plurality of B-mode images. That is, the step S17 is a process of moving the scanning plane such that the correlation value between the B-mode image and the sample image becomes the maximum.

On the other hand, when the maximum value of the correlation values is smaller than the predetermined threshold, the process moves to step S 18. In the step S 18, the transmission/reception control section 21 moves the position of the scanning plane with respect to the second direction L2 to the center of the range of scanning. The state where the maximum value of the correlation values is not equal to or larger than the predetermined threshold is supposed to be the state where the treatment instrument 50 is not protruded from the treatment instrument insertion port 17. Therefore, in the step S 18, the scanning plane is moved to a position where the scanning plane and the central axis of the treatment instrument insertion port 17 substantially agree with each other.

Then, the scanning plane position optimization process is terminated, and the process returns to the step S03 in FIG. 8. In the step S03, the counter value t is reset to be zero (t = 0). Then, in the step S04, an ultrasound beam is scanned in the first direction L1 on the scanning plane whose position has been determined in the scanning plane position optimization process in the step S02.

Next, in the step S05, the B-mode image calculation section 22 generates a B-mode image based on the result of the scanning in the step S04. Then, in the step S06, the generated B-mode image is outputted to the image display apparatus 4. According to the process, the B-mode image is displayed on the image display apparatus 4.

Next, in the step S07, it is determined whether or not an instruction for terminating the observation using the B-mode image has been inputted by operating the ultrasound observation switch 25. When the instruction for terminating the observation using the B-mode image has been inputted, the operation is stopped.

On the other hand, when the instruction for terminating the observation using B-mode image is not inputted, the process moves to the step S08, and one is added to the counter value t (t = t+1). Then, in the step S09, it is determined whether or not the counter value t is smaller than a predetermined threshold Th.

When the counter value t is smaller than the predetermined threshold Th, the process returns to step S04, and the scanning of ultrasound beam and the generation of B-mode image are repeated. When the counter value t reaches the predetermined Th, the process returns to the step S02, and the scanning plane position optimization process is performed.

That is, in the present embodiment, after the scanning of the ultrasound beam and the generation of the B-mode image are repeated by a predetermined number of times Th on the scanning plane located at a certain position with respect to the second direction L2, the scanning plane position optimization process is performed.

In the scanning plane position optimization process, the position of the scanning plane is determined such that the correlation value between the B-mode image and the sample image becomes the maximum, that is, the shape of the treatment instrument 50 appears most clearly on the B-mode image.

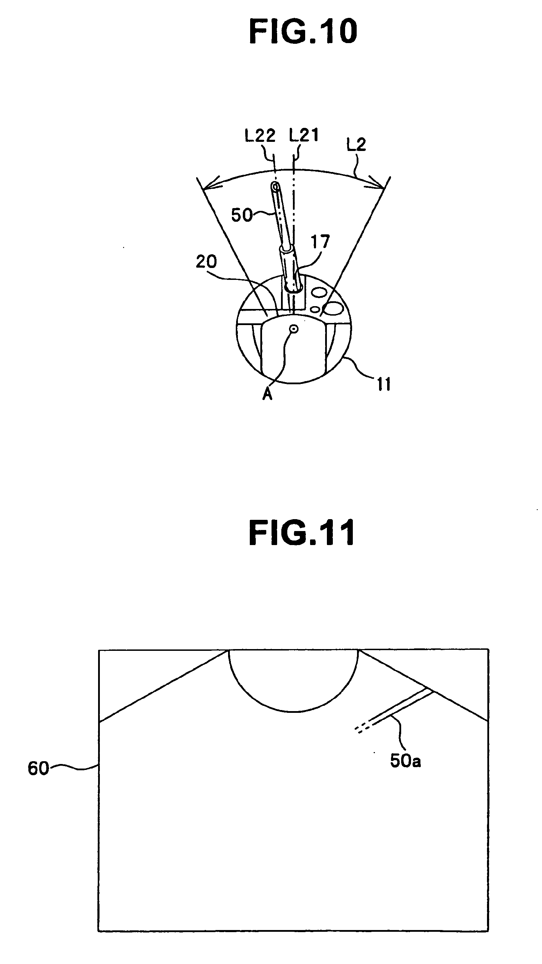

For example, as shown in FIG. 10, in a case where the protruding direction of the treatment instrument 50 protruded from the treatment instrument insertion port 17 is inclined with respect to the central axis of the treatment instrument insertion port 17, if the scanning plane is remained fixed at a position (position shown by the two-dot-chain line L21 in FIG. 10) where the scanning plane substantially agrees with the central axis of the treatment instrument insertion port 17 as in a conventional configuration, observation of the treatment instrument 50 on the B-mode image 60 becomes difficult as shown in FIG. 11.

According to the ultrasound observation apparatus 1 in the present embodiment, even in the case where the protruding direction of the treatment instrument 50 is thus inclined, the scanning plane position optimization process is performed, and thereby the scanning plane is automatically moved to the position (position shown by the two-dot-chain line L22 in FIG. 10) where the echo pattern of the treatment instrument 50 shows the shape of treatment instrument 50 most clearly.

In addition, the scanning plane position optimization process is periodically performed in a period during which the observation using the B-mode image is continued. Therefore, even if the treatment instrument 50 is deviated from the scanning plane in the middle of the treatment performed on a subject, the scanning plane automatically moves, which allows the treatment instrument 50 to be captured again in the B-mode image 60 without the need for the user to intentionally perform any operation.

That is, according to the ultrasound observation apparatus 1 of the present embodiment, it is possible to continue excellent observation of the treatment instrument 50 on the B-mode image 60 without performing any cumbersome operation.

### (Second Embodiment)

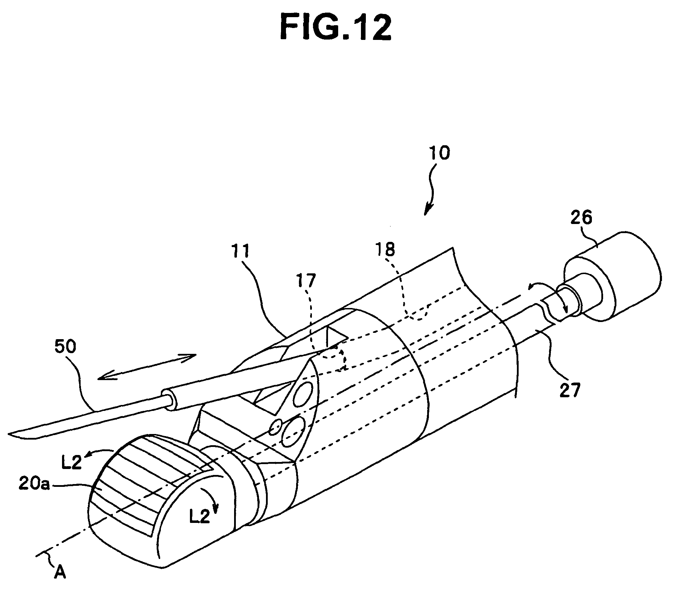

The second embodiment of the present invention will be described below with reference to FIGS. 12 and-13. The ultrasound observation apparatus according to the present embodiment is different from the first embodiment in the method of scanning of ultrasound beam performed by the ultrasound probe portion 20. Only the points different from the first embodiment will be described below, and the same components as those in the first embodiment are attached with the same reference numerals and description thereof will be omitted accordingly.

As shown in FIG. 12, the ultrasound probe portion 20a of the present embodiment has a configuration of what is called a convex scanning type, in which a plurality of ultrasound transducers are aligned in a row in a substantially circular arc shape. A plurality of ultrasound transducers constituting the ultrasound probe portion 20a are aligned such that scanning of an ultrasound beam can be performed in substantially sector shape on the plane parallel to the insertion axis A of the insertion portion 10. That is, the ultrasound probe portion 20a is configured to enable electronic scanning of an ultrasound beam in the first direction L1.

In addition, the ultrasound probe portion 20a is disposed so as to be oscillatable around the axis parallel to the insertion axis A of the insertion portion 10. That is, the ultrasound probe portion 20a is configured to enable mechanical scanning of an ultrasound beam in the second direction L2 by oscillating in the second direction L2.

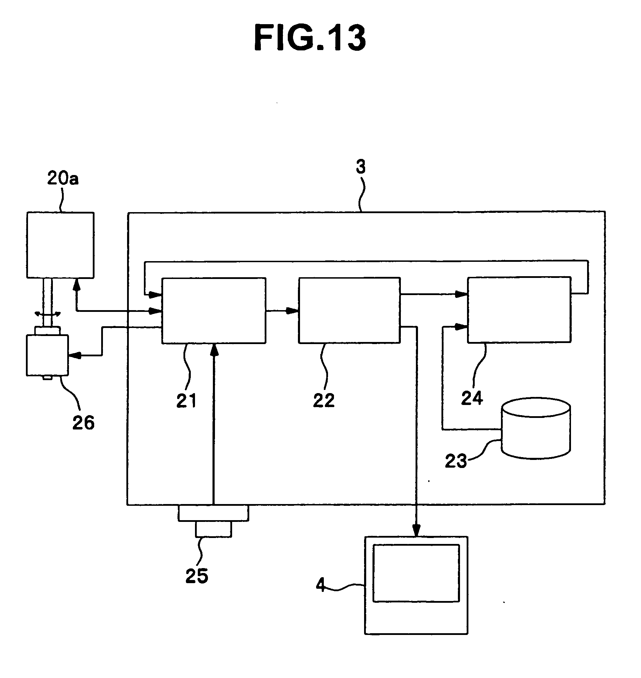

The ultrasound probe portion 20a is connected to an electric motor 26 provided in the operation portion 30 via a flexible shaft 27 inserted through the insertion portion 10. The ultrasound probe portion 20a oscillates around the axis parallel to the insertion axis A by the drive force generated by the electric motor 26. As shown in FIG. 13, the electric motor 26 is electrically connected to the transmission/reception control section 21, and the operation of the electric motor 26 is controlled by the transmission/reception control section 21.

As described above, the ultrasound probe portion 20a according to the present embodiment can perform two-dimensional scanning of the ultrasound beam by combining the electronic scanning and the mechanical scanning. In addition, the direction of the scanning of the ultrasound beam performed by the ultrasound probe portion 20a is controlled by the transmission/reception control section similarly as in the first embodiment.

It is needless to say that the same effects as those in the first embodiment can be obtained also by the present embodiment described above.

According to the above-described embodiment, it is possible to provide an ultrasound observation apparatus for observing a situation of a treatment using a treatment instrument in a subject, which is capable of continuing excellent observation of the treatment instrument.

The present invention is not limited to the above-described embodiments, and can be modified as needed without departing from the gist of invention which can be read from claims and the whole specification. An ultrasound observation apparatus modified as such is also included in the technical range of the present invention.

As described above, the present invention is suitable for an ultrasound observation apparatus which observes a situation of a treatment using a treatment instrument in a subject.

## Claims

1. An ultrasound observation apparatus (1) for generating a B-mode image of inside a subject by scanning an ultrasound beam on a scanning plane in the subject, the ultrasound observation apparatus (1) comprising:
an ultrasound probe portion (20) capable of two-dimensional scanning of an ultrasound beam by changing a transmission/reception direction of the ultrasound beam in a first direction and in a second direction;
a transmission/reception control section (21) for controlling the transmission/reception direction of the ultrasound beam by the ultrasound probe portion (20);
a B-mode image calculation section (22) for generating the B-mode image based on a result of scanning of the ultrasound beam in the first direction; and
a storage section (23) storing a sample image,
**characterized by**
a correlation calculation section (24) for calculating a correlation value between the B-mode image and the sample image, wherein
the transmission/reception control section (21) is configured for moving the scanning plane in the second direction such that the correlation value becomes the maximum, and
the sample image has been determined according to a shape of a treatment instrument (50) in a case where a central axis of the treatment instrument (50) for performing a treatment on the subject and the scanning plane agree with each other.

2. The ultrasound observation apparatus (1) according to claim 1, wherein
the ultrasound probe portion (20) is provided to an ultrasound endoscope (2) which can be introduced into a subject, and
the ultrasound endoscope (2) includes a treatment instrument insertion port (17) for allowing the treatment instrument (50) to protrude into a range of the scanning of the ultrasound beam by the ultrasound probe portion (20).

3. The ultrasound observation apparatus (1) according to claim 2, wherein
the ultrasound probe portion (20) is arranged such that the scanning plane is parallel to a central axis of the treatment instrument insertion port (17) and the second direction is along a plane which is perpendicular to the scanning plane,
the transmission/reception control section (21) is configured forming the scanning plane to a plurality of locations in the second direction with the treatment instrument (50) protruded from the insertion port (17),
the B-mode image calculation section (22) is configured generating a plurality of B-mode images acquired in a state where the scanning plane is positioned at the plurality of locations in the second direction,
the correlation calculation section (24) is configured for calculating correlation values between the plurality of B-mode images and the sample image, and
the transmission/reception control section (21) is configured for moving the scanning plane to a position where the maximum correlation value is acquired among the correlation values of the plurality of B-mode images.

4. The ultrasound observation apparatus (1) according to claim 3, wherein,
when the maximum value of the correlation values is smaller than a predetermined threshold, the transmission/reception control section (21) moves the scanning plane to a center position of a range of scanning in the second direction.

5. The ultrasound observation apparatus (1) according to clam 1, wherein
the transmission/reception control section (21) is configured for periodically moving the scanning plane such that the correlation value becomes the maximum.

6. The ultrasound observation apparatus (1) according to claim 5, wherein,
the transmission/reception control section (21) is configured for periodically moving the scanning plane after a predetermined number of times of generation of the B-mode image by the B-mode image calculation section (22) and calculation of the correlation value by the correlation calculation section (24).

7. The ultrasound observation apparatus (1) according to claim 1, wherein
the sample image is an image showing a shape and a size of the treatment instrument (50) in the B-mode image in a case where the scanning plane and the central axis of the treatment instrument (50) agree with each other.

8. The ultrasound observation apparatus (1) according to claim 7, wherein
the sample image is an image stored in the storage section (23) by designating an echo pattern of the treatment instrument (50) in the B-mode image generated by the B-mode image calculation section (22).

9. The ultrasound observation apparatus (1) according to claim 1, wherein
the ultrasound probe portion (20) includes a plurality of ultrasound transducers which are aligned in matrix and can be driven individually, and the ultrasound probe portion (20) is configured to electronically perform the two-dimensional scanning of the ultrasound beam by controlling a driving timing of each of the ultrasound transducers.

10. The ultrasound observation apparatus (1) according to claim 1, wherein
the ultrasound probe portion (20) includes a plurality of ultrasound transducers arranged in a row in a circular arc shape, and the ultrasound probe portion (20) is configured to perform the two-dimensional scanning of the ultrasound beam by performing electronic scanning of the ultrasound beam in the first direction by controlling a driving timing of each of the ultrasound transducers, and by performing mechanical scanning of the ultrasound beam in the second direction.

11. The ultrasound observation apparatus (1) according to claim 1, wherein
the sample image shows a shape of a puncture needle, a biopsy forceps, or a cytological brush in the B-mode image.

## Patentansprüche

1. Ultraschall-Beobachtungsgerät (1) zum Erzeugen eines B-Mode-Bilds des Inneren eines Patienten durch Bewegen eines Ultraschallstrahls auf einer Abtastebene in dem Patienten, wobei das Ultraschall-Beobachtungsgerät (1) umfasst:
einen Ultraschallsondenbereich (20), der einen Ultraschallstrahl durch Ändern einer Übertragungs-/Empfangsrichtung des Ultraschallstrahls in einer ersten Richtung und einer zweiten Richtung zweidimensional zu bewegen vermag;
einen Übertragungs-/Empfangssteuerabschnitt (21) zum Steuern der Übertragungs-/Empfangsrichtung des Ultraschallstrahls durch den Ultraschallsondenbereich (20);
einen B-Mode-Bildberechnungsabschnitt (22) zum Erzeugen des B-Mode-Bilds auf der Grundlage eines Ergebnisses des Bewegens des Ultraschallstrahls in der ersten Richtung; und
einen Speicherabschnitt (23), der ein Probenbild speichert,
**gekennzeichnet durch**
einen Korrelationsberechnungsabschnitt (24) zum Berechnen eines Korrelationswerts zwischen dem B-Mode-Bild und dem Probenbild, wobei
der Übertragungs-/Empfangssteuerabschnitt (21) dazu ausgebildet ist, die Abtastebene in der zweiten Richtung so zu bewegen, dass der Korrelationswert maximal wird, und
das Probenbild nach Maßgabe einer Form eines Behandlungsinstruments (50) in einem Fall bestimmt worden ist, in dem eine Mittelachse des Behandlungsinstruments (50) zur Durchführung einer Behandlung an dem Patienten und die Abtastebene miteinander übereinstimmen.

2. Ultraschall-Beobachtungsgerät (1) nach Anspruch 1, wobei
der Ultraschallsondenbereich (20) an einem Ultraschallendoskop (2) vorgesehen ist, das in einen Patienten eingeführt werden kann, und
das Ultraschallendoskop (2) einen Behandlungsinstrument-Einführungsanschluss (17) beinhaltet, um zu erlauben, dass das Behandlungsinstrument (50) in einen Bereich des Bewegens des Ultraschallstrahls durch den Ultraschallsondenbereich (20) vorsteht.

3. Ultraschall-Beobachtungsgerät (1) nach Anspruch 2, wobei
der Ultraschallsondenbereich (20) so angeordnet ist, dass die Abtastebene parallel zu einer Mittelachse des Behandlungsinstrument-Einführungsanschlusses (17) ist und die zweite Richtung entlang einer Ebene führt, die senkrecht zur Abtastebene ist,
der Übertragungs-/Empfangssteuerabschnitt (21) dazu ausgebildet ist, die Abtastebene an mehrere Stellen in der zweiten Richtung zu bewegen, wobei das Behandlungsinstrument (50) aus dem Einführungsanschluss (17) vorsteht,
der B-Mode-Bildberechnungsabschnitt (22) dazu ausgebildet ist, mehrere B-Mode-Bilder zu erzeugen, die in einem Zustand erfasst werden, in dem die Abtastebene an den mehreren Stellen in der zweiten Richtung positioniert ist,
der Korrelationsberechnungsabschnitt (24) dazu ausgebildet ist, Korrelationswerte zwischen den mehreren B-Mode-Bildern und dem Probenbild zu berechnen, und
der Übertragungs-/Empfangssteuerabschnitt (21) dazu ausgebildet ist, die Abtastebene an eine Position zu bewegen, an der der maximale Korrelationswert unter den Korrelationswerten der mehreren B-Mode-Bilder erfasst wird.

4. Ultraschall-Beobachtungsgerät (1) nach Anspruch 3, wobei,
wenn der maximale Wert der Korrelationswerte kleiner als ein vorgegebener Schwellwert ist, der Übertragungs-/Empfangssteuerabschnitt (21) die Abtastebene an eine Mittelposition eines Bereichs zum Abtasten in der zweiten Richtung bewegt.

5. Ultraschall-Beobachtungsgerät (1) nach Anspruch 1, wobei
der Übertragungs-/Empfangssteuerabschnitt (21) dazu ausgebildet ist, die Abtastebene periodisch so zu bewegen, dass der Korrelationswert maximal wird.

6. Ultraschall-Beobachtungsgerät (1) nach Anspruch 5, wobei
der Übertragungs-/Empfangssteuerabschnitt (21) dazu ausgebildet ist, die Abtastebene nach einer vorgegebenen Anzahl von Malen der Erzeugung des B-Mode-Bilds durch den B-Mode-Bildberechnungsabschnitt (22) und die Berechnung des Korrelationswerts durch den Korrelationsberechnungsabschnitt (24) periodisch zu bewegen.

7. Ultraschall-Beobachtungsgerät (1) nach Anspruch 1, wobei
das Probenbild ein Bild ist, das eine Form und eine Größe des Behandlungsinstruments (50) im B-Mode-Bild in einem Fall zeigt, in dem die Abtastebene und die Mittelachse des Behandlungsinstruments (50) miteinander übereinstimmen.

8. Ultraschall-Beobachtungsgerät (1) nach Anspruch 7, wobei
das Probenbild ein Bild ist, das im Speicherabschnitt (23) durch Bezeichnen eines Echomusters des Behandlungsinstruments (50) im B-Mode-Bild, das durch den B-Mode-Bild-Berechnungsabschnitt (22) erzeugt wird, gespeichert ist.

9. Ultraschall-Beobachtungsgerät (1) nach Anspruch 1, wobei
der Ultraschallsondenbereich (20) mehrere Ultraschallwandler beinhaltet, die in einer Matrix ausgerichtet sind und einzeln angesteuert werden können, und der Ultraschallsondenbereich (20) dazu ausgebildet ist, das zweidimensionale Bewegen des Ultraschallstrahls durch Steuern einer Ansteuerzeiteinteilung von jedem der Ultraschallwandler elektronisch durchzuführen.

10. Ultraschall-Beobachtungsgerät (1) nach Anspruch 1, wobei
der Ultraschallsondenbereich (20) mehrere Ultraschallwandler beinhaltet, die in einer Reihe in Kreisbogenform angeordnet sind, und der Ultraschallsondenbereich (20) dazu ausgebildet ist, das zweidimensionale Bewegen des Ultraschallstrahls durch Ausführen eines elektronischen Abtastens des Ultraschallstrahls in der ersten Richtung durch Steuern einer Ansteuerzeiteinteilung von jedem der Ultraschallwandler und durch Ausführen eines mechanischen Abtastens des Ultraschallstrahls in der zweiten Richtung durchzuführen.

11. Ultraschall-Beobachtungsgerät (1) nach Anspruch 1, wobei
das Probenbild die Form einer Punktionskanüle, einer Biopsiezange oder eines zytologischen Pinsels im B-Mode-Bild zeigt.

## Revendications

1. Appareil d'observation ultrasonore (1) pour générer une image en mode B de l'intérieur d'un sujet par balayage d'un faisceau ultrasonore sur un plan de balayage dans le sujet, l'appareil d'observation ultrasonore (1) comprenant :
une partie de sonde ultrasonore (20) apte à un balayage bidimensionnel d'un faisceau ultrasonore en changeant un sens d'émission/réception du faisceau ultrasonore dans un premier sens et dans un deuxième sens ;
une section de commande d'émission/réception (21) pour commander le sens d'émission/réception du faisceau ultrasonore par la partie de sonde ultrasonore (20) ;
une section de calcul d'image en mode B (22) pour générer l'image en mode B sur la base d'un résultat de balayage du faisceau ultrasonore dans le premier sens ; et
une section de stockage (23) stockant une image échantillon,
**caractérisé par**
une section de calcul de corrélation (24) pour calculer une valeur de corrélation entre l'image en mode B et l'image échantillon, dans lequel
la section de commande d'émission/réception (21) est configurée pour déplacer le plan de balayage dans le deuxième sens de telle sorte que la valeur de corrélation devient le maximum, et
l'image échantillon a été déterminée en fonction d'une forme d'un instrument de traitement (50) dans un cas où un axe central de l'instrument de traitement (50) pour exécuter un traitement sur le sujet et le plan de balayage sont en accord l'un avec l'autre.

2. Appareil d'observation ultrasonore (1) selon la revendication 1, dans lequel
la partie de sonde ultrasonore (20) est prévue sur un endoscope ultrasonore (2) qui peut être introduit dans un sujet, et
l'endoscope ultrasonore (2) inclut un orifice d'insertion (17) d'instrument de traitement pour permettre que l'instrument de traitement (50) fasse saillie dans une plage du balayage du faisceau ultrasonore par la partie de sonde ultrasonore (20).

3. Appareil d'observation ultrasonore (1) selon la revendication 2, dans lequel
la partie de sonde ultrasonore (20) est agencée de telle manière que le plan de balayage est parallèle à un axe central de l'orifice d'insertion (17) d'instrument de traitement et le deuxième sens est le long d'un plan qui est perpendiculaire au plan de balayage,
la section de commande d'émission/réception (21) est configurée pour déplacer le plan de balayage jusqu'à une pluralité d'emplacements dans le deuxième sens avec l'instrument de traitement (50) en saillie hors de l'orifice d'insertion (17),
la section de calcul d'image en mode B (22) est configurée pour générer une pluralité d'images en mode B acquises dans un état où le plan de balayage est positionné au niveau de la pluralité d'emplacements dans le deuxième sens,
la section de calcul de corrélation (24) est configurée pour calculer des valeurs de corrélation entre la pluralité d'images en mode B et l'image échantillon, et
la section de commande d'émission/réception (21) est configurée pour déplacer le plan de balayage jusqu'à une position où la valeur de corrélation maximum est acquise parmi les valeurs de corrélation de la pluralité d'images en mode B.

4. Appareil d'observation ultrasonore (1) selon la revendication 3, dans lequel
lorsque la valeur maximum des valeurs de corrélation est plus petite qu'un seuil prédéterminé, la section de commande d'émission/réception (21) déplace le plan de balayage jusqu'à une position centrale d'une plage de balayage dans le deuxième sens.

5. Appareil d'observation ultrasonore (1) selon la revendication 1, dans lequel
la section de commande d'émission/réception (21) est configurée pour déplacer périodiquement le plan de balayage de telle manière que la valeur de corrélation devient le maximum.

6. Appareil d'observation ultrasonore (1) selon la revendication 5, dans lequel
la section de commande d'émission/réception (21) est configurée pour déplacer périodiquement le plan de balayage après un nombre de fois prédéterminé de génération de l'image en mode B par la section de calcul d'image en mode B (22) et de calcul de la valeur de corrélation par la section de calcul de corrélation (24).

7. Appareil d'observation ultrasonore (1) selon la revendication 1, dans lequel
l'image échantillon est une image montrant une forme et une taille de l'instrument de traitement (50) dans l'image en mode B dans un cas où le plan de balayage et l'axe central de l'instrument de traitement (50) sont en accord l'un avec l'autre.

8. Appareil d'observation ultrasonore (1) selon la revendication 7, dans lequel
l'image échantillon est une image stockée dans la section de stockage (23) en désignant un motif d'écho de l'instrument de traitement (50) dans l'image en mode B générée par la section de calcul d'image en mode B (22).

9. Appareil d'observation ultrasonore (1) selon la revendication 1, dans lequel
la partie de sonde ultrasonore (20) inclut une pluralité de transducteurs ultrasonores qui sont alignés en matrice et peuvent être pilotés individuellement, et la partie de sonde ultrasonore (20) est configurée pour exécuter électroniquement le balayage bidimensionnel du faisceau ultrasonore en commandant une synchronisation de pilotage de chacun des transducteurs ultrasonores.

10. Appareil d'observation ultrasonore (1) selon la revendication 1, dans lequel
la partie de sonde ultrasonore (20) inclut une pluralité de transducteurs ultrasonores agencés en une rangée sous une forme d'arc de cercle, et la partie de sonde ultrasonore (20) est configurée pour exécuter le balayage bidimensionnel du faisceau ultrasonore en exécutant un balayage électronique du faisceau ultrasonore dans le premier sens en commandant une synchronisation de pilotage de chacun des transducteurs ultrasonores, et en exécutant un balayage mécanique du faisceau ultrasonore dans le deuxième sens.

11. Appareil d'observation ultrasonore (1) selon la revendication 1, dans lequel
l'image échantillon montre une forme d'une aiguille de piquage, d'une pince de biopsie, ou d'une brosse cytologique dans l'image en mode B.
